Europäisches Patentamt

(19) European Patent Office

Office européen des brevets

(11) Publication number: **0 005 266**
**B1**

(12) **EUROPEAN PATENT SPECIFICATION**

(45) Date of publication of patent specification: **26.01.83**

(51) Int. Cl.³: **A 61 M 1/03**

(21) Application number: **79101337.8**

(22) Date of filing: **02.05.79**

(54) Hemodialysis apparatus.

(30) Priority: **03.05.78 IT 341578**

(43) Date of publication of application:
**14.11.79 Bulletin 79/23**

(45) Publication of the grant of the patent:
**26.01.83 Bulletin 83/4**

(84) Designated Contracting States:
**BE DE GB IT NL SE**

(56) References cited:
**DE - A - 1 566 657**
**DE - A - 1 566 661**
**DE - A - 2 005 808**
**GB - A - 1 406 133**
**US - A - 3 388 803**
**US - A - 3 868 154**
**US - A - 3 979 284**

(73) Proprietor: **Bonomini, Vittorio**
**Via Siepelunga 30**
**Bologna (IT)**
(73) Proprietor: **Ruggeri, Giovanni**
**Via Boves 4**
**Bologna (IT)**

(72) Inventor: **Bonomini, Vittorio**
**Via Siepelunga 30**
**Bologna (IT)**
Inventor: **Ruggeri, Giovanni**
**Via Boves 4**
**Bologna (IT)**

(74) Representative: **Gudel, Diether, Dr. et al,**
**Patentanwälte Dipl.-Ing. P. Wirth Dr. Schmied-**
**Kowarzik Dipl.-Ing. G. Danneberg Dr. P. Weinhold**
**Dr. D. Gudel Grosse Eschenheimer Strasse 39**
**D-6000 Frankfurt/Main (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Hemodialysis apparatus

The present invention relates to a preferably portable hemodialysis apparatus of the type comprising a first circuit for blood and a second circuit for ultrafiltration and dialysis liquid, the exchange between the blood and the dialysis liquid taking place in a dialyzer through a semipermeable membrane, in each of said first and second circuits pump means being provided and first means being provided for slectively connecting the output side of the pump means for said dialysis liquid either with the inlet of said dialyzer or with an ultrafiltrate collection station.

The importance of the hemodialysis in order to ensure the depuration of blood in an evenly increasing number of patients from impurities or waste materials and toxic substances is well known and does not require extended comment: it is enough to mention that, whereas the resolutive therapy of chronic renal insufficiency can be, as it is, the kidney transplantation, drawn from a donor (either living or dead), without however the certainty of the successful result of such an operation, the probabilities of keeping the patient alive by a periodical hemodialysis treatment are much higher, as is confirmed by the worldwide statistics.

It is thus clear that hemodialysis is the indispensable treatment, both in the case of patients waiting for kidney transplantation, and in the case of patients for whom, owing to whatever reason, such an operation is not possible or to be foreseen within a short time.

The hemodialysis by itself is a matter exchange process between two liquid phases (one of which is the blood, whereas the other is a solution having a controlled concentration of some predetermined components); the exchange takes place through a membrane of well known type, adapted to permit the passage of molecules of some size, normally molecules having size not greater than a given size and therefore than a given molecular weight.

In the course of the hemodialysis the patient is deprived of a predetermined amount (not negligible) of water and of impurities and toxic substances which pass into the dialysis liquid; otherwise stated, thus, in the hemodialysis, besides the true dialysis, there takes place also a phase, important as well for the purpose of the treatment, of ultrafiltration of the water contained in the blood.

The standard hemodialysis apparatus, normally installed in hospitals for the service of a number of patients, generally comprise an extracorporeal circuit of the blood and a circuit of the dialysis liquid: the blood, as drawn by means of a pump, from a blood vessel of the patient, passes through the dialyzer, in which the countercurrent exchange with the dialysis liquid takes place through the semipermeable membrane, to return to a vein of the patient; at the outlet of the dialyzer there are provided control and safety devices, having the purpose of ensuring that any blood clots or air bubbles cannot, by error or accident, reenter with the blood flow in the corporeal circuit (with the readily understandable consequences), these devices further ensuring the compatibility of the reentering pressure with the blood pressure existing the patient's body.

In turn the circuit of the dialysis liquid comprises a high capacity tank (150 to 300 litres), which is fed with demineralized water and in which a solution is prepared having a predetermined and desired concentration of some components.

Such a solution, suitably heated to a predetermined temperature, consistent with that of the blood in the body circuit (otherwise there are possible drawbacks and problems of cardiocirculatory nature, apart from an uneasy feeling of bodily discomfort, mainly of intense cold of the patient) passes through the dialyzer in countercurrent flow with respect to the blood, to be thereafter directly discharged into the sewer.

In the hemodialysis treatment (normally having a duration of about 4 hours and a repetition frequency of three times per week), there can be located two phases, namely that of true dialysis, in which toxic substances and impurities (normally small molecules) pass through the semipermeable membrane from the blood to the dialysis liquid, and that of the ultrafiltration, in which a pressure difference between the blood circuit and the circuit of the dialysis liquid, more precisely a reduced pressure in the circuit of the dialysis liquid, causes the blood content of water to be reduced by a predetermined amount.

It is now necessary to point out that, besides the true clinical problems of the hemodialysis treatment, relating to the patient's survival, a few others exist, as hereinafter explained:

1) The patient is permanently bound to a dialysis center, without possibility of departing for times greater than 48 hours.

2) Any damage to the hemodialysis apparatus involves a real danger of death for all the patients depending on the said apparatus.

3) The apparatus, besides being very costly and cumbersome, must be provided with special auxiliary parts, such as for instance a weighing or balance bed, capable of indicating during the operation the progressive reduction of weight of the patients due to the water elimination by ultrafiltration, which occurs simultaneously with the dialysis.

4) The costs not only of construction, but also and mainly of operation of these hemodialysis apparatus (specialized personnel permanently attending to the operation thereof, enormous water and electric power consumption, etc.) are to date very high and, if account is taken of the fact that the number of patients needing this treatment is constantly increasing, it is readily appreciated that in the long run their existence for the community shall become intolerable. In recent times, some attempts have been made and reported for providing portable hemodialysis apparatus adapted to permit the patient to enjoy some movement freedom, although not complete, allowing at the same time the hemodialysis to be carried out at home, the costs being markedly reduced and (which is a very important advantage) rendering the patient independent from the hemodialysis hospital center.

Among these attempts particularly noteworthy, in order to better understand the present invention, is the so-called wearable artificial kidney as described and illustrated in the paper by W. J. Kolff, "Concepts, Critics and Comments: Wide and Varied (a Festschrift in Honor of David Rose)", November 1976.

This apparatus comprises a hollow fiber dialyzer in which the exchange between the patient's blood, as drawn by a single needle system, connected via a single needle unit and an anti-regurgitation valve to the patient. The dialysate is produced by providing a negative pressure in the dialysate conpartment which obtains fluid by ultrafiltration, the dialysate volume being designed at about 500 mls.

The dialysate is regenerated via a small regeneration system (based on a replaceable absorption unit) on each pass through the dialyzer.

The circulaton of both the blood and the dialysate takes place thanks to a special pump, comprising two ventricles which are alternately compressed by means of an actuator driven by a small 12 Volt DC motor. During the diastolic phase the blood is drawn from the patient's body, through the said single needle system, a valve being provided to prevent blood from being drawn from the extracorporeal circuit, and into the ventricle, whereas, during the systolic phase, the blood is ejected from the pumping ventricle and flows through the dialyzer, to return to the patient through the same single needle system; devices are to be provided for safety, e.g. against air embolism.

As regards the dialysate side of the system, the dialyzing fluid is supplied by an accumulator, whereas the ultrafiltrate being generated is collected into a special bag for a later disposal.

The wearable module is intermittently connected to a 20 liter tank, receiving the fluid as ejected by the pump and feeding the absorption unit, during this time both the dialysate accumulator and the ultrafiltrate collector being not operative.

As a matter of fact the patient can be disconnected from the 20 liter tank for not more than 15 minutes, which means that for approximately two thirds of the dialysis time the patient must be connected, even through long connection lines, to the 20 liter tank. According to the data reported in said paper, the wearable artificial kidney permits the dialysis to be carried out at home or wherever a 12 volt electrical source is available, the dialysis time, both for the true dialysis and for the ultrafiltration being not less than 5 hours, since the ultrafiltration occurs at a rate of about 700 mls/hour. Furthermore, another problem of the above apparatus is that of the flow limitations as resulting from the intermittent operation of the pump and by the use of the single needle system (the latter in turn being necessary due to the pump type), which can be selected between 0 and 250 mls per minute, whereas flow rates of 300 mls and more per minute are more suitable for an optimum hemodialysis.

US—A—3 979 284 describes a portable hemodialysis apparatus with the features as cited above. This apparatus does not have any means for connecting the inlet of the pump means for the second circuit by choice either directly with the dialyzer or with a supply tank or dialysis liquid.

The main purpose of the present invention is that of providing a portable · hemodialysis apparatus, in which the blood to be depurated is continuously drawn from the patient's body, and continuously injected again in a different point, whereas the dialysis solution is continuously circulated and regenerated, it being a rather small volume. Another purpose of the present invention is to provide a portable hemodialysis apparatus, in which the treatment, besides being carried out in whatever place a source of electrical power is available, has a duration not greater than that of the standard hospital operation.

The invention, therefore, is characterized by second means being provided for selectively connecting the input side of the pump means for said dialysis liquid with the outlet of the dialyzer either through a supply tank of dialysis liquid or directly via a shunt line, a pressure meter being arranged in said shunt line upstream of the pump means for said dialysis liquid.

In the first configuration, in which the suction side of the pump is directly connected to the outlet side of the dialyzer, a reduced pressure on the dialysis liquid side can be established in the dialyzer thus permitting the water contained in the blood to be extracted by ultrafiltration, this water being discharged by the pump into a graduated collecting bag, which permits the amount or weight of extracted water to be readily estimated.

In the second configuration, in which the suction side of the pump is directly connected to the outlet of a tank containing a water solution having a predetermined content- of selected components, thus forming the dialysis liquid, the tank is provided with means for the control and adjustment of the temperature of the dialysis liquid, and the outlet of the pump is directly in communication with the inlet of the dialyzer, an absorption filter being provided between the outlet of the dialyzer and the inlet of the tank to permit the dialysis liquid after the matter exchange in the dialyzer, to be regenerated by absorption of the toxic substances.

The treatment of the blood comprises a first step in which said first configuration is operative and only ultrafiltration takes place until a predetermined amount of water is removed from the blood of the patient, the second step being the true dialysis, in which the dialysis circuit is in the said second configuration.

The supply tank preferably is removably attachable to said casing to permit the patient to be free from the said tank for a limited time.

The several features and advantages of the present invention shall appear from the following detailed description, having illustrative but not limitative meaning, of a preferred embodiment, with reference to the enclosed drawings in which:

Fig. 1 is a schematic view of the apparatus of the present invention;

Fig. 2 is a perspective view of the tank for the dialysis solution;

Fig. 3 is a perspective view of the portable unit to be worn by the patient undergoing the dialysis;

Fig. 4 is a perspective view of an auxiliary device for the unit of Fig. 3 and

Fig. 5 is a perspective view of the pump used for the circulation of the blood as well as of the dialysis liquid.

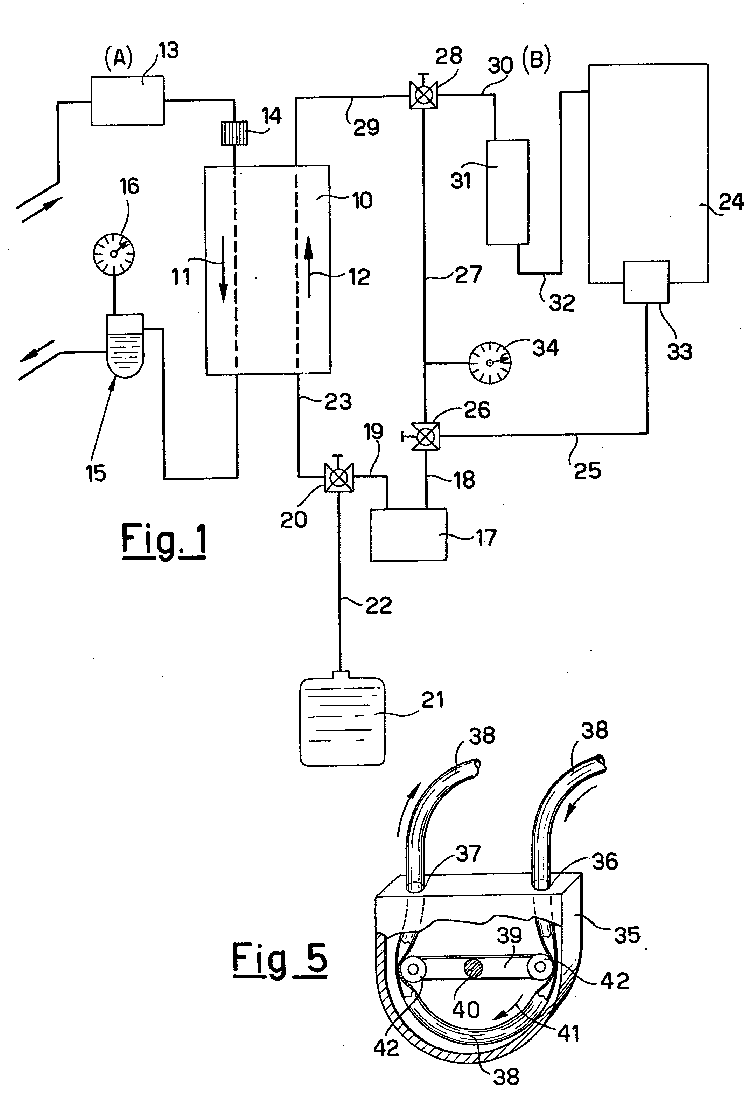

Referring firstly to Fig. 1, there are shown two circuits, namely one for the blood (A) and one for the dialysis liquid and for the ultrafiltration (B).

Reference 10 indicates the dialyzer, which can be of a well known type; for instance the dialyzer can be of the type in which the blood runs along a cavity formed between two semipermeable membranes, whereas the dialysis liquid flows countercurrently on the outer surface of both membranes.

According to another preferred embodiment, the dialyzer comprises a bundle of capillary fibres, the inside passage of which is run by the blood, whereas the dialysis liquid flows countercurrently along the outer surfaces of the fibres. Of course, every other type of dialyzer known in the art and of suitable size, provided that it permits the desired exchange between the blood and the dialysis liquid, can be used as well.

The blood is circulated in the dialyzer 10 in the direction indicated by the arrow 11, whereas the dialysis liquid circulates in the direction as indicated by the arrow A, it comprises a pump 13, as hereinafter more detailedly described, a filter 14, containing a material suitable for the hemoperfusion, for instance special activated carbon, having the purpose of removing toxic substances from the blood before it enters the dialyzer, thus increasing the efficacy of the depuration. The filter 14 is not compulsory and is of the replaceable type. Before the blood reenters the body circuit of the patient, it is passed through a device 15, of a type per se known, having the purpose of preventing blood clots and air bubbles to be entrapped with the blood flow; furthermore the pressure measuring device 16 permits the pressure of the blood being fed back to the patient's vein to be adjusted to a suitable value.

The blood is drawn either from a vein or from an artery, whereas the return to the patient's body takes place always through a vein.

The part (B) of the apparatus, namely the circuit of the dialysis liquid comprises a pump 17, of the same type as the pump 13, having a suction tube 18 and a delivery tube 19. The latter, through a suitable two-way valve 20, can be put into communication either with a collecting bag 21 (via the connecting line 22), having metering lines permitting the liquid contained thereinto to be measured, or with the inlet side of the dialyzer 10, via the connecting line 23.

The tube 18 is connected by means of the valve 26, either to the supply tank 24, through a line 25, or to a shunt line 27, which in turn, through the two-way valve 28 can be directly connected to the outlet of the dialyzer (via the connecting line 29).

The valve 28 is connected through the line 30 to a filtering replaceable cartridge 31, positioned upstream of the tank 24, and having the purpose of regenerating the dialysis liquid by absorbing the toxic substances extracted from the blood in the dialyzer.

The outlet of the cartridge 31 is connected via the line 32 to the tank 24, whereas by the reference 33 a device is shown having the purpose of controlling the temperature of the dialysis liquid being drawn by the pump 17 to a predetermined value. The cartridge 31 is charged with an absorption material, such as active carbon, serving for the stated purpose and per se well known in the art.

In the shunt line 27 a pressure gauge 34 is inserted, in order to measure the reduced pressure or vacuum generated by the pump 17, in the dialyzer 10 during the ultrafiltration phase.

Turning now to the Fig. 5 the head or operative part of the pumps 13 and 17 is shown; more particularly, this head comprises a casing 35 in the upper part of which two holes 36 and 37 are formed for the entry and the exit of the tube 38, whereas the lower part has a curved shape, to which the tube 38 is thoroughly adhered. The actuator 39 of the pump head is mounted to the outlet shaft 40 of an electric motor (not shown) the actuator being rotated in the sense of the arrow 41. Both ends of the actuator 39 are proved with idle rollers 42, the distance of the rollers from the axis of the shaft 40 being selected so as to have the tube 38, which is of suitable collapsible material (i.e. plastic material), throttled between the roller and the adjacent inner surface of the casing 35.

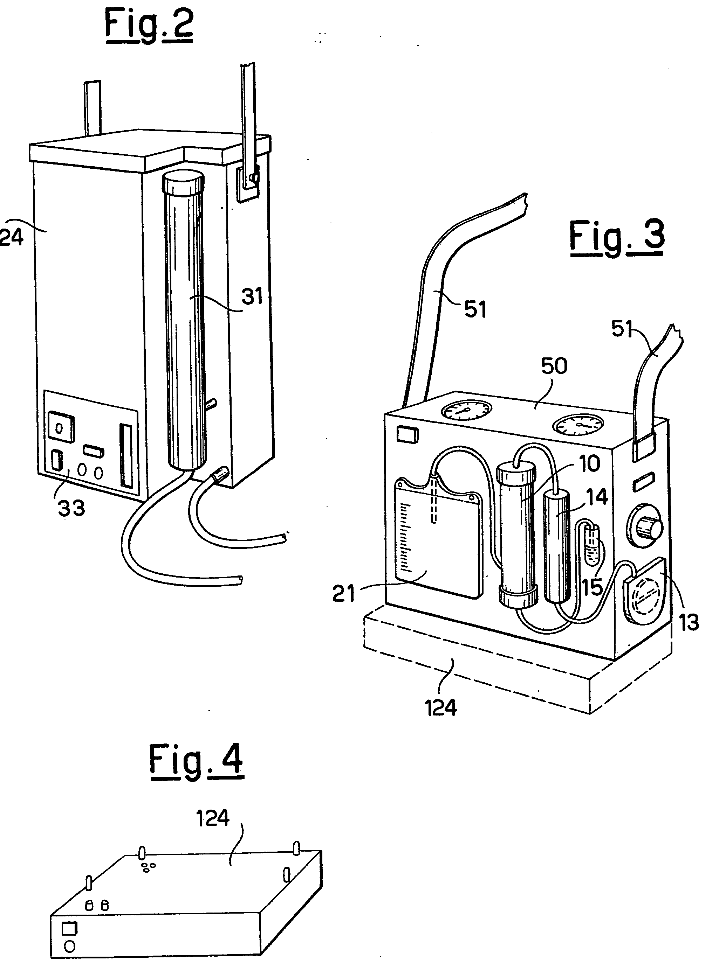

Of course, the rotation of the actuator 39 in the direction of the arrow 41 causes the pumping effect to occur. Turning now to the Figures 2 and 3, a preferred embodiment of the portable hemodialysis apparatus is shown comprising a casing 50, which can be attached to the patient's chest by means of the straps or brace 51; the casing 50 enclosing all the previously described components, apart from the tank 24, the temperature control and adjusting device 33 and the filtering cartridge 31, which are formed as a unit and shown in the Fig. 2.

The two pumps 13 and 17 are mounted at the two opposite sides of the casing 50 and their motors are driven by any suitable electric source, such as the mains, a battery or like, provided that the motors of the pumps are adapted for these electrical supplies.

Instead of the valves 20, 26 and 28, quick connecting and disconnecting joints might be provided, according to the two operation phases of the apparatus.

The auxiliary component shown in Fig. 4 and adapted to be positioned as shown by dashed lines in Fig. 3, permits the tank 24 to be temporarily detached, to permit the patient, wearing only the portable unit, to be completely free from too much weight (if account is taken that the tank 24 normally has a capacity of about 20 litres).

The collecting bag 21 is normally removed after the ultrafiltration phase, and possibly substituted for by a filter cartridge identical to the filter 31, the latter being in this case omitted from the unit of Fig. 2.

The latter is normally independently mounted, possibly on castor wheels, and can be quickly connected to the unit of Fig. 3, to give the configuration of Fig. 1.

Turning now to the operation of the portable apparatus of the present invention, as already mentioned two phases can be identified, namely the ultrafiltration and the dialysis.

A. Ultrafiltration

In this phase the blood is drawn by the pump 13, while a reduced pressure is established in the dialyzer, on the dialysis liquid side, by means of the pump 17, connected by the shunt line 27 and the line 29, to the outlet of the dialyzer 10, The delivery side of the pump 17 is connected, through the lines 19 and 22, to the collecting bag 21.

Of course, the valves 20, 26 and 28 must be suitably controlled to provide the above described circuit.

B. Dialysis

Upon the ultrafiltration period being completed, the valve 20 is switched to connect the delivery side of the pump 17, through the lines 19 and 23, to the inlet of the dialyzer, whereas the valves 26 and 28 are switched to exclude the shunt line 27, the circuit of the dialysis liquid including now the filter cartridge 31, the tank 24 and the temperature control device 33. Then the dialysis takes place for a predetermined time, at the end of which the portable unit is disconnected fro the patient's blood vessels, and is ready for the next operation.

The apparatus of the present invention has been tested in vivo, on voluntary patients, both awaiting the transplantation of the kidney and normally undergoing the hemodialysis to depurate their blood.

Apart from the clinical results as hereinafter shortly résuméd, the patients have constantly indicated a well-being status, apparently better than that stated with the standard hemodialysis apparatus.

As regards the therapeutical results, it is to be pointed out that one of the very important features of the present apparatus is that of permitting the ultrafiltration to be carried out as the first phase, thus permitting the water to be removed from the patient to be drawn from the very beginning, without any significant cardiocirculatory modifications of status of the organism.

Furthermore such a first ultrafiltration permits part of the toxic substances to be removed together with the water thus making easier the subsequent dialysis.

The practical tests resulted in a treatment time not longer than that of the fixed standard apparatus or even shorter, due to the ultrafiltration being carried out as the first step. The dialysis treatment as carried out with the apparatus of the present invention (I) has been compared either with the standard apparatus (III), and with the wearable artificial kidney (II) (as average values calculated from the data reported in the paper of Kolff):

TABLE 1
Material removed during the first hour of ultrafiltration

|  | I | II | III |
|---|---|---|---|
| $H_2O$ mls. | 2 500 | 700 | 2 500 |
| urea mg | 300 | 112 | 300 |
| uric acid mg | 95 | 37 | 98 |
| creatinine | 18 | 7.5 | 19 |

TABLE 2
Material removed during the first hour of dialysis

|  | I | II | III |
|---|---|---|---|
| urea mg | 1 500 | 1 200 | 1 700 |
| uric acid | 550 | 450 | 550 |
| creatinine mg | 1 000 | 850 | 1 050 |

The above results confirm that the portable apparatus of the present invention permit the hemodialysis to be carried out, with performance rates at least comparable with those of the standard hospital treatment, but with the outstanding advantages as previously shortly pointed out.

In this connection it is furthermore to be pointed out that the apparatus of the present invention, due to the complete separation between the ultrafiltration and the dialysis phases, permits not only the standard hemodialysis to be carried out, but also the ultrafiltration alone, as well as the dialysis alone to be carried out, by merely suitable adjusting the circuit from the side of the dialysis liquid.

Of course, the results of the present invention are mainly due to the adoption of the particular pumps, both on the blood side and on the dialysis liquid side, since no danger exists of damage to the pump during the ultrafiltration, namely when the reduced pressure is to be established in the dialyzer and no liquid at least initially passes through the pump 17.

Claims

1. A, preferably portable, hemodialysis apparatus of the type comprising a first circuit (A) for blood and a second circuit (B) for ultrafiltration and dialysis liquid, the exchange between the blood and the dialysis liquid taking place in a dialyzer (10) through a semipermeable membrane, in each of said first and second circuits pump means (13, 17) being provided, and first means (20) being provided for selectively connecting the output side of the pump means (17) for said dialysis liquid either with the inlet of said dialyzer (10) or with an ultrafiltrate collection station (12), characterized by, second means (26, 28) being provided for selectively connecting the input side of the pump means (17) for said dialysis liquid with the outlet of the dialyzer (10) either through a supply tank (24) of dialysis liquid or directly via a shunt line (27), a pressure meter (34) being arranged in said shunt line (27) upstream of the pump means (17) for said dialysis liquid.

2. A hemodialysis apparatus according to claim 1, characterized by a switchable two-way valve (20) for the selective connection of said output side of said pump means (17) for said dialysis liquid to either the inlet of said dialyzer (10) or to said collection station (21).

3. A hemodialysis apparatus according to claim 1, characterized by two-way valves (26, 28) for the connection of said input side of said pump means (17) for said dialysis liquid to the outlet of said dialyzer (10) through said shunt line (27), the switching of said two-way valves (26, 28) permitting said input side to communicate with said outlet either through said shunt line (27) or through said supply tank (24).

4. A hemodialysis apparatus according to claim 1, characterized by temperature control and adjustment means (33) for said supply tank (24).

5. A hemodialysis apparatus according to claim 1, characterized by a filter (14) for the absorption of toxic substances arranged upstream of said dialyzer (10) in said first circuit (A) and a control and safety device (15, 16) arranged downstream of said dialyzer (10) in said first circuit (A) to prevent the entry of blood clots and air into the patient's body, and to control the blood pressure.

6. A hemodialysis apparatus according to claim 1, characterized by said supply tank (24) and said filter (14) in said dialysis liquid forming a separate unit, the remainder of the apparatus being mounted in a unitary casing (50) for attachment to the patient's body.

7. A hemodialysis apparatus according to claim 6, characterized by said supply tank (24) being removably attachable to said casing.

## Revendications

1. Appareil d'hémodialyse, de préférence portatif, du type comprenant un premier circuit (A) pour le sang et un deuxième circuit (B) pour du liquide d'ultrafiltration et de dialyse, l'échange entre le sang et le liquide de dialyse ayant lieu dans un dialyseur (10) au travers d'une membrane semi-perméable, un moyen de pompage (13, 17) étant prévu dans chacun des premier et deuxième circuits, un premier moyen (20) étant prévu pour relier de manière sélective le côté sortie du moyen de pompage (17) du liquide de dialyse soit avec l'entrée du dialyseur (10) soit avec une station de récolte du produit d'ultrafiltration (21), caractérisé par un deuxième moyen (26, 28) qui est prévu pour relier de manière sélective le côté entrée du moyen de pompage (17) du liquide de dialyse avec la sortie du dialyseur (10), soit par l'intermédiaire d'un réservoir d'alimentation (24) en liquide de dialyse, soit directement par l'intermédiaire d'une ligne de dérivation (27), un dispositif de mesure de pression (24) étant agencé dans la ligne de dérivation (27) en amont du moyen de pompage (17) du liquide de dialyse.

2. Appareil d'hémodialyse suivant la revendication 1, caractérisé par une soupape à deux voies (20) commutable pour relier de manière sélective le côté sortie du moyen de pompage (17) du liquide de dialyse soit à l'entrée du dialyseur (10) soit à la station de récolte (21).

3. Appareil d'hémodialyse suivant la revendication 1, caractérisé par des soupapes à deux voies (26, 28) pour relier le côté entrée du moyen de pompage (17) du liquide de dialyse à la sortie du dialyseur (10) par la ligne de dérivation (27, la commutation de ces soupapes à deux voies (26, 28) permettant de faire communiquer le côté entrée susdit avec ladite sortie soit par l'intermédiaire de la ligne de dérivation (27) soit par l'intermédiaire du réservoir d'alimentation (24).

4. Appareil d'hémodialyse suivant la revendication 1, caractérisé par un moyen de réglage et d'ajustement de la température (33) pour le réservoir d'alimentation (24).

5. Appareil d'hémodialyse suivant la revendication 1, caractérisé par un filtre (14) d'absorption de substances toxiques, qui est agencé en amont du dialyseur (10) dans le premier circuit susdit (A) et par un dispositif de réglage et de sécurité (15, 16) agencé en aval du dialyseur (10) dans le premier circuit (A) pour empêcher l'entrée de caillots de sang et d'air dans le corps du patient et pour régler la pression sanguine.

6. Appareil d'hémodialyse suivant la revendication 1, caractérisé en ce que le réservoir d'alimentation (24) et le filtre (31) du liquide de dialyse forment une unité séparée, le restant de l'appareil étant monté dans un boîtier unitaire (50) pour être attaché au corps du patient.

7. Appareil d'hémodialyse suivant la revendication 6, caractérisé en ce que le réservoir d'alimentation peut être attaché de manière détachable au boîtier.

## Patentansprüche

1. Vorzugsweise tragbare Hämodialyseeinrichtung mit einem ersten Kreislauf (A) für Blut und mit einem zweiten Kreislauf (B) für eine Ultrafiltrations- und Dialyseflüssigkeit, wobei der Austausch zwischen dem Blut und der Dialyseflüssigkeit in einem Dialysator (10) über eine semipermeable Membran stattfindet, wobei ferner in beiden Kreisläufen Pumpen (13, 17) vorgesehen sind und wobei erste Mittel (20) vorgesehen sind, um wahlweise die Ausgangsseite der Pumpe (17) für die Dialyseflüssigkeit entweder mit dem Einlaß des Dialysators (10) zu verbinden oder mit einer Sammelstation (21) für Ultrafiltrat, dadurch gekennzeichnet, daß zweite Mittel (26, 28) vorgesehen sind, um die Eingangsseite der Pumpe (17) für die Dialyseflüssigkeit mit dem Ausgang des Dialysators (10) entweder über einen Behälter (24) der Dialyseflüssigkeit oder direkt über eine Zweigleitung (27) zu verbinden, in der stromauf der Pumpe (17) für die Dialyseflüssigkeit ein Druckmeßgerät (34) angeordnet ist.

2. Hämodialyseeinrichtung nach Anspruch 1, dadurch gekennzeichnet, daß ein umschaltbares Zwei-Wege-Ventil (20) für die wahlweise Verbindung der Ausgangsseite der Pumpe (17) für die Dialyseflüssigkeit entweder mit dem Einlaß des Dialysators (10) oder mit der Sammelstation (21) vorgesehen ist.

3. Hämodialyseeinrichtung nach Anspruch 1, dadurch gekennzeichnet, daß Zwei-Wege-Ventile (26, 28) für die Verbindung der Eingangsseite der Pumpe (17) für die Dialyseflüssigkeit mit dem Ausgang des Dialysators (10) über die Zweigleitung (27) vorgesehen sind, wobei die Schaltung der Zwei-Wege-Ventile (26, 28) es gestattet, daß die Eingangsseite mit dem Ausgang entweder über die Zweigleitung (27) oder über den Behälter (24) verbunden wird.

4. Hämodialyseeinrichtung nach Anspruch 1, dadurch gekennzeichnet, daß eine Einrichtung zur Temperatursteuerung für den Behälter (24) vorgesehen ist.

5. Hämodialyseeinrichtung nach Anspruch 1, dadurch gekennzeichnet, daß zur Absorption toxischer Substanzen stromauf des Dialysators (10) im ersten Kreislauf (A) ein Filter (14) vorgesehen ist und daß stromab des Dialysators (10) im ersten Kreislauf (A) eine Steuerungs- und Sicherheitseinrichtung (15, 16) angeordnet ist, mit der die Zufuhr von Blutklümpchen und Luft in den Körper des Patienten verhindert und der Blutdruck kontrolliert wird.

6. Hämodialyseeinrichtung nach Anspruch 1, dadurch gekennzeichnet, daß der Behälter (24) und das Filter (14) in der Dialyseflüssigkeit eine getrennte Einheit ausbilden und daß die anderen Bauteile der Einrichtung an einem Gehäuse (50) zur Befestigung am Körper des Patienten montiert sind.

7. Hämodialyseeinrichtung nach Anspruch 6, dadurch gekennzeichnet, daß der Vorratsbehälter (24) lösbar am Behälter befestigt ist.

0 005 266

(A) 13

(B) 28 30

14

29

16

10

11 12

31 24

27

23 34

19 26

20 32

18 25

33

22

17

21

<u>Fig.1</u>

38 38

37 36

39

35

<u>Fig 5</u>

42

40 41

42

38

0 005 266

# Fig.2

24

31

33

# Fig.3

51

50

51

10

14

21

15

13

124

# Fig.4

124

2